# FASCICULE DE BREVET EUROPEEN

(11) **EP 4 212 196 B1**
(45) Date de publication et mention de la délivrance du brevet: **10.07.2024**
(21) Numéro de dépôt: 22213555.0
(22) Date de dépôt: 14.12.2022
(51) Int. Cl.: A61M 16/00, A61B 5/08, A61B 5/097

(54) **VENTILATEUR MÉDICAL AVEC MONITORAGE D'UNE RÉANIMATION CARDIO-PULMONAIRE**
MEDIZINISCHES BEATMUNGSGERÄT MIT ÜBERWACHUNG DER KARDIOPULMONALEN WIEDERBELEBUNG
MEDICAL VENTILATOR WITH MONITORING OF CARDIOPULMONARY RESUSCITATION

(30) Priorité: 13.01.2022 FR 2200260
(43) Date de publication de la demande: 19.07.2023
(73) Titulaire: Air Liquide Medical Systems, 92182 Antony Cedex (FR)
(72) Inventeur: LESIMPLE, Arnaud, 92182 Antony Cedex (FR); PROUVEZ, Nathan, 92182 Antony Cedex (FR); RICHARD, Jean-Christophe, 92182 Antony Cedex (FR); COURCENET, Jean-Philippe, 92182 Antony Cedex (FR); BROC, Alexandre, 92182 Antony Cedex (FR)
(74) Mandataire: Air Liquide

(56) Documents cités:
- EP-A1- 3 639 735
- EP-A1- 4 026 578
- US-A1- 2020 215 293

## Description

L'invention concerne un ventilateur médical comprenant des moyens de monitorage des gaz « expirés » par un patient, pendant une réanimation cardio-pulmonaire ou RCP, permettant d'opérer un diagnostic clinique en temps réel du patient après analyse d'un signal de teneur en CO₂ contenu dans les gaz sortant des poumons du patient.

L'arrêt cardiaque ou arrêt cardio-respiratoire est une cause fréquente de mortalité chez l'homme. Un arrêt cardiaque se traduit par un arrêt du fonctionnement du coeur, c'est-à-dire un arrêt du pouls, et donc aussi de l'envoi du sang vers le cerveau et le reste du corps humain, en particulier les autres organes vitaux.

Pour être efficace, une prise en charge de l'arrêt cardiaque ou réanimation cardio-pulmonaire ou RCP doit comprendre un massage cardiaque accompagné d'une ventilation du patient, c'est-à-dire une assistance respiratoire du patient. Une RCP de qualité permet d'augmenter considérablement les chances de survie du patient.

Plus précisément, le massage cardiaque permet de suppléer la pompe cardiaque pour faire circuler le sang vers et dans les différents organes. Il doit être pratiqué le plus rapidement possible sur la personne en arrêt cardiaque. Un massage cardiaque comprend une alternance de compressions et décompressions thoraciques, à une fréquence d'environ 100 à 120 coups/minutes avec une profondeur de compression entre 4 et 6 cm.

Par ailleurs, la ventilation assistée délivrée au patient par le personnel soignant permet de favoriser les échanges gazeux pulmonaires, à savoir l'apport d'oxygène (O₂) et la sortie ou lavage du CO₂ contenu dans le sang.

La ventilation assistée peut être dispensée au moyen d'un insufflateur de gaz manuel, c'est-à-dire un ballon auto-remplisseur à valve unidirectionnelle ou BAVU, comme enseigné par EP-A-3645093, EP-A-3488890 et EP-A-3884983. Un tel BAVU est typiquement utilisé par les unités d'urgence mobiles, tels les unités du SAMU ou des pompiers par exemple.

Alternativement, dans des situations plus complexes ou en milieu hospitalier par exemple, on peut aussi utiliser un appareil ou ventilateur d'assistance respiratoire, plus simplement appelé « ventilateur médical », servant à dispenser le gaz d'assistance au patient, tel de l'air ou de l'air enrichi en O₂. De tels ventilateurs médicaux sont par exemple enseignés par EP-A-3218036, EP-A-3299055, EP-A-3218035 et EP-A-3216479.

Toutefois, mettre en oeuvre une ventilation efficace pendant une RCP, permettant d'augmenter les chances de survie de la personne en arrêt cardiaque, i.e. du patient, n'est pas chose aisée. En effet, une ventilation excessive ou, à l'inverse, insuffisante peut affecter négativement la circulation sanguine du patient, donc nuire au succès de la RCP. Il est donc primordial d'opérer un monitorage permettant un suivi de la qualité de la ventilation et de la circulation délivrées pendant une RCP.

Certains documents, comme US-A-2020/215293, EP-A-3511043, EP-A-3510924 et EP-A-3639735, proposent de suivre, i.e. monitorer et afficher, la quantité de CO₂ présent dans les gaz sortant des poumons du patient, aussi appelés les gaz 'expirés', et de s'en servir pour s'assurer du bon déroulé de la ventilation, par exemple pour déterminer un retour à une circulation sanguine spontanée du patient ou RACS.

Or, actuellement, les ventilateurs médicaux affichent au mieux des courbes de suivi, par exemple de la teneur en CO₂, mais c'est au personnel soignant, c'est-à-dire aux secouristes ou médecins par exemple, de les interpréter pour pouvoir éventuellement adapter ensuite la ventilation délivrée au patient subissant la RCP. On comprend aisément que cela engendre des risques pour le patient, notamment en cas de mauvaise interprétation des courbes conduisant à un choix de ventilation inadaptée.

Le document EP-A-4026578 (non publié ; relevant de l'Art. 54(3) CBE) recommande de réaliser un monitorage des gaz sortant ou expirés par un patient pendant une RCP, telle la teneur en CO₂, et de proposer un diagnostic clinique du patient. Toutefois, ce document ne préconise pas d'intégrer les éléments permettant d'opérer ces monitorage et diagnostic au sein d'un ventilateur médical mais dans un boitier autonome conçu pour se fixer à un ventilateur ou à un insufflateur manuel de type BAVU. On comprend que devoir recourir à un boitier autonome n'est pas idéal car cela oblige à gérer plusieurs appareils, peut conduire à de mauvais raccordements et/ou à une perte de temps lors des accordements, voire même à des pertes du boitier autonome, ou à d'autres problèmes liés.

Dans ce contexte, le problème est de proposer un ventilateur médical amélioré permettant non seulement d'opérer un monitorage efficace des gaz sortant ou expirés par un patient pendant une RCP, en particulier de la teneur en CO₂, mais aussi de proposer un diagnostic clinique, en temps réel, du patient ventilé, à partir des mesures de CO₂ opérées et ce, sans avoir recours à un boitier autonome ou analogue.

La solution de l'invention concerne un ventilateur médical adapté à la mise en oeuvre d'une ventilation assistée pendant une réanimation cardio-pulmonaire (RCP) d'un patient, comprenant :
- une source de gaz,
- un circuit ventilatoire comprenant une branche inspiratoire alimentée en gaz par la source de gaz, et une branche expiratoire venant se raccorder l'une et l'autre à une pièce de jonction,
- une interface respiratoire raccordée à la pièce de jonction et en communication fluidique avec le circuit ventilatoire,
- des moyens de pilotage à microprocesseur, i.e. un ou plusieurs microprocesseurs,
- des moyens de mesure de CO₂ configurés pour opérer des mesures successives de teneur en CO₂ dans le flux gazeux et fournir lesdites mesures de teneur en CO₂ aux moyens de pilotage, lesdits moyens de pilotage traitant lesdites mesures de teneur en CO₂, et
- des moyens d'affichage commandés par lesdits moyens de pilotage,
dans lequel :
- les moyens de pilotage sont en outre configurés pour :
   i) traiter au moins une partie des valeurs de teneurs en CO₂ pour établir au moins un diagnostic de l'état ventilatoire du patient choisi parmi une fermeture des voies aériennes, une distension thoracique et une ventilation cible, et
   iii) commander un affichage dudit au moins un diagnostic d'état ventilatoire sur les moyens d'affichage,
- et les moyens d'affichage sont configurés pour afficher ledit au moins un diagnostic d'état ventilatoire.

De plus, selon l'invention :
- la source de gaz comprend une turbine motorisée commandée par les moyens de pilotage, et
- les moyens de pilotage sont en outre configurés pour :
   i) déterminer au moins une recommandation d'action à mener par le personnel soignant en fonction dudit au moins un diagnostic d'état ventilatoire, et
   ii) commander un affichage de ladite au moins une recommandation d'action à mener sur les moyens d'affichage, et
- les moyens d'affichage sont configurés pour afficher ladite au moins une recommandation d'action à mener.

Dans le cadre de l'invention :
- par « mesure » ou « valeur » d'une grandeur donnée, en particulier de pression, de débit ou de teneur en CO₂, on entend une valeur mesurée ou un signal représentatif d'une valeur mesurée.
- par « état ventilatoire », on entend une indication de qualité de la ventilation assistée opérée sur le patient en arrêt cardiaque au moyen de l'appareil de ventilation assistée, c'est-à-dire un ventilateur médical, selon l'invention.
- par « état circulatoire », on entend une indication de qualité de la réanimation cardiopulmonaire (RCP) avec massage cardiaque (i.e. succession de compressions thoraciques et de relâchements) pratiquée par un ou plusieurs secouristes, i.e. du personnel soignant, sur le patient en arrêt cardiaque.
- les termes « moyens pilotage » et « moyens de traitement de données » sont considérés équivalents et englobent des moyens informatiques à microprocesseur(s) permettant d'opérer notamment des calculs, des comparaisons, des commandes, des pilotages... ou tout autre traitement informatisé ou similaire.

Selon le mode de réalisation considéré, le ventilateur médical peut comprendre l'une ou plusieurs des caractéristiques suivantes :
- il comprend en outre des moyens de mesure de pression pour déterminer au moins une pression du gaz et pour fournir ladite au moins une valeur de pression mesurée aux moyens de pilotage.
- il comprend en outre des moyens de détermination de débit pour déterminer au moins une valeur de débit de gaz correspondant au débit du gaz dans le circuit ventilatoire et fournir ladite au moins une valeur de débit de gaz auxdits moyens de pilotage.
- les moyens de pilotage sont configurés pour traiter lesdites au moins une valeur de pression et de débit mesurées pour établir ledit au moins un diagnostic de l'état ventilatoire du patient ou pour confirmer un diagnostic d'état ventilatoire obtenu par traitement, i.e. analyse, des valeurs de teneurs en CO₂.
- les moyens de mesure de CO₂ comprennent un capteur de CO₂ agencé entre l'interface respiratoire et la pièce de jonction, et est relié aux moyens de pilotage via une liaison filaire.
- le capteur de CO₂ peut être agencé directement sur le flux gazeux ou flux principal (*main stream* en anglais) ou sur un flux gazeux dérivé, c'est-à-dire en dérivation (*side stream* en anglais)
- les moyens d'affichage sont en outre configurés pour afficher une courbe de CO₂, de préférence en temps réel, à partir des mesures de teneur en CO₂ traitées par les moyens de pilotage.
- les moyens de pilotage sont en outre configurés pour établir un diagnostic d'état circulatoire du patient à partir d'au moins une partie des mesures traitées et pour commander un affichage dudit diagnostic d'état circulatoire sur les moyens d'affichage.
- les moyens d'affichage sont configurés pour afficher ledit diagnostic d'état circulatoire.
- les moyens de pilotage sont configurés pour traiter au moins une partie des mesures de teneurs en CO₂ et de pression et/ou de débit de gaz pour établir ledit au moins un diagnostic d'état ventilatoire et/ou d'état circulatoire.
- les moyens de pilotage sont en outre configurés pour déterminer au moins une recommandation d'action à mener par le personnel soignant en fonction des diagnostics d'état ventilatoire et d'état circulatoire.
- ladite au moins une recommandation d'action à mener est choisie parmi une augmentation de la pression positive, une baisse du volume courant ou une continuité de la ventilation, et/ou une augmentation ou une diminution de la force de compression du massage cardiaque, et une augmentation ou une diminution de la fréquence du massage cardiaque.
- il comprend en outre des moyens de mémorisation pour mémoriser les diagnostics d'état ventilatoire, les diagnostics d'état circulatoire et les recommandations d'action à mener.
- les moyens de pilotage sont en outre configurés pour aller retrouver au sein des moyens de mémorisation, le diagnostic d'état ventilatoire, le diagnostic d'état circulatoire et/ou la recommandation d'action à mener à afficher sur les moyens d'affichage.
- les moyens de pilotage sont configurés pour établir au moins un diagnostic de l'état ventilatoire ou circulatoire du patient ou pour proposer une recommandation d'action à mener en allant retrouver au sein des moyens de mémorisation, le diagnostic d'état ventilatoire, le diagnostic d'état circulatoire et/ou la recommandation d'action à mener à afficher sur les moyens d'affichage correspondant aux valeurs de teneurs en CO₂, de débit et/ou de pression ayant été traitées par lesdits moyens de pilotage.
- il comprend des moyens d'alimentation électrique pour fournir, directement ou indirectement, du courant électrique au moins aux moyens de pilotage, aux moyens d'affichage et à la turbine motorisée.
- la pièce de jonction est une pièce en Y.
- les moyens d'affichage comprennent un écran d'affichage, de préférence à affichage en couleurs.
- les moyens de mesure de CO₂ comprennent un capteur de CO₂.
- les moyens de mesure de pression comprennent un capteur de pression.
- les moyens de détermination de débit comprennent un capteur de débit massique ou un capteur de pression différentielle.
- les moyens de pilotage sont configurés pour établir et commander un affichage d'un diagnostic de l'état ventilatoire et d'un diagnostic de l'état circulatoire du patient.
- les moyens de mesure de pression sont agencés de manière à déterminer la pression du gaz régnant dans le passage de gaz interne reflétant la pression au sein du circuit ventilatoire, qui reflète celle dans les voies aériennes du patient.
- les moyens de pilotage sont configurés pour commander un affichage sur les moyens d'affichage, d'une courbe en temps réel de CO₂ obtenue à partir de plusieurs mesures successives de teneurs en CO₂ successives traitées, c'est-à-dire un profil de CO₂.
- les moyens d'affichage sont configurés pour afficher ladite courbe en temps réel de CO₂.
- les moyens de pilotage sont configurés pour traiter des mesures de teneurs en CO₂ et des mesures de pression et de débit de gaz pour établir au moins un diagnostic d'état ventilatoire et au moins un diagnostic d'état circulatoire, c'est-à-dire tout ou partie des mesures opérées pour l'un ou plusieurs de ces diagnostics.
- les moyens de pilotage sont configurés pour établir un diagnostic de l'état circulatoire du patient comprenant une indication de la manière dont s'effectue le massage cardiaque, par exemple un massage trop fort ou trop faible (i.e. insuffisant), ou un massage trop rapide ou trop lent, ou autre.
- le diagnostic d'état ventilatoire et/ou d'état circulatoire sont obtenus à partir de données mémorisées pouvant se présenter sous différentes formes telles que valeurs cibles, algorithmes obtenus à partir de données cliniques ou autres etc.....
- les moyens de pilotage sont en outre configurés pour déterminer au moins une recommandation d'action à mener par un personnel soignant en fonction dudit au moins un diagnostic d'état ventilatoire et/ou d'état circulatoire établi, c'est-à-dire une recommandation « ventilatoire » et/ou une recommandation « circulatoire ».
- les moyens de pilotage sont en outre configurés pour commander un affichage de ladite au moins une recommandation d'action à mener sur les moyens d'affichage, c'est-à-dire la recommandation « ventilatoire » et/ou la recommandation « circulatoire ».
- les moyens d'affichage sont configurés pour afficher la ou les recommandations d'action à mener, c'est-à-dire la recommandation « ventilatoire » et/ou la recommandation « circulatoire ».
- la recommandation d'action à mener, typiquement la recommandation « ventilatoire », est choisie parmi une augmentation de la pression positive, une baisse du volume courant et une continuité de la ventilation (i.e. maintien sans changement).
- la recommandation d'action à mener, typiquement la recommandation « circulatoire », comprend une modification d'un ou plusieurs paramètres qualitatifs reflétant la qualité d'un massage cardiaque choisis parmi une augmentation de la force de compression du massage cardiaque, une diminution de la force de compression du massage cardiaque, une augmentation de la fréquence du massage cardiaque et une diminution de la fréquence du massage cardiaque.
- les moyens de détermination de débit comprennent un capteur de débit massique ou un capteur de pression différentielle, i.e. à perte de charge.
- il comprend un circuit de gaz interne servant à l'acheminement du gaz dans la carcasse du ventilateur entre la sortie de la source de gaz, typiquement une turbine motorisée, et l'entrée de la branche inspiratoire du circuit ventilatoire.
- la turbine motorisée comprend une entrée d'aspiration permettant d'aspirer de l'air ambiant.
- il comprend des moyens d'alimentation en oxygène permettant d'apporter de l'oxygène supplémentaire destiné à être mélangé à l'air aspiré par la turbine, le mélange air/oxygène étant opéré en amont, en aval ou dans la turbine.
- les moyens d'alimentation en oxygène comprennent une ligne d'alimentation en oxygène et un connecteur d'entrée servant à raccorder fluidiquement la ligne d'alimentation en oxygène à une source d'oxygène externe, telle une bouteille d'oxygène sous pression ou une conduite d'amenée d'oxygène débouchant par exemple au niveau d'une prise d'oxygène murale ou autre.
- le circuit de gaz interne comprend un conduit ou passage de gaz.
- les moyens de détermination de teneur en CO₂ comprennent un capteur de CO₂, i.e. un dispositif permettant de déterminer une teneur, une quantité ou une concentration en CO₂.
- le capteur de CO₂ est un dispositif de spectrométrie infra-rouge.
- le capteur de pression est configuré pour convertir la pression ou les variations de pression en tension ou variations de tension, ou en intensité ou variation d'intensité électrique, appelée(s) « mesure(s) de pression ».
- le capteur de débit massique est relié électriquement aux moyens de pilotage pour leur fournir une ou des mesures de débit, de préférence via un (ou des) câble ou fil électrique ou analogue.
- les moyens de détermination de débit sont configurés pour mesurer le débit de gaz circulant dans un sens uniquement ou dans les deux sens au sein du circuit ventilatoire, c'est-à-dire le gaz allant vers le patient et entrant dans ses poumons et le gaz sortant des poumons du patient.
- les moyens d'affichage comprennent un écran à affichage numérique, notamment un écran tactile.
- les moyens de pilotage comprennent (au moins) une carte électronique.
- la (au moins une) carte électronique comprend le (ou les) microprocesseur, de préférence un microcontrôleur.
- le microprocesseur met en oeuvre un ou plusieurs algorithmes.
- la carte électronique comprend les moyens de stockage de données.
- la carte électronique est alimentée en courant électrique.
- les moyens de fourniture de courant électrique comprennent au moins une batterie, notamment rechargeable. La batterie peut être extractible ou non du ventilateur, de préférence elle est extractible pour pouvoir être rechargée, via un chargeur dédié.
- alternativement, les moyens de fourniture de courant électrique comprennent une liaison secteur (110/220V), par exemple un cordon muni d'une prise de raccordement, permettant d'alimenter le ventilateur pour fournir du courant électrique aux composants nécessitant du courant électrique pour fonctionner et/ou à la batterie interne pour la recharger lorsqu'elle est complètement ou partiellement vide et/ou a été utilisée par exemple.
- les moyens de fourniture de courant électrique comprennent éventuellement un transformateur de courant pour réduire la tension et/ou l'intensité du courant, en particulier lorsqu'il provient du secteur (110/220 V).
- les moyens de pilotage sont en outre configurés pour commander un affichage sur les moyens d'affichage, d'un volume de gaz calculé à partir du débit de gaz ayant été déterminé ou mesuré, par exemple par calcul de l'intégrale du débit de gaz (i.e. du débit en temps réel) sur une période de temps donnée, par exemple une durée de 0.5 à 10 secondes.
- les moyens de pilotage sont configurés pour commander un affichage, sur les moyens d'affichage, d'au moins une valeur de volume de gaz ou de teneur en CO₂, par exemple une valeur maximale (Vmax) de CO₂, ou une (ou des) représentation graphique d'au moins une desdites valeurs de volume et/ou de teneur en CO₂, par exemple une représentation graphique de type barre-graphe ou autre, de préférence la valeur maximale (Vmax) de CO₂ est déterminée sur un intervalle de temps donné, par exemple une durée de 3 à 10 secondes.
- la carcasse est rigide, par exemple en polymère.
- la carcasse comprend l'écran d'affichage, par exemple il affleure à la surface d'une des faces de la carcasse.
- l'écran d'affichage est connecté électriquement aux moyens de pilotage, en particulier au microprocesseur.
- les moyens de stockage de données sont une mémoire de stockage, par exemple de type EEPROM ou autre.
- les moyens de stockage de données connecté électriquement aux moyens de traitement de données, en particulier au microprocesseur.
- il comprend en outre des moyens d'alarme (sonore et/ou visuelle) configurés pour déclencher une alarme, notamment lorsque l'on détecte une pression des voies aériennes trop importante, un débranchement patient, un volume insufflé trop important, des voies aériennes fermées ou autres.
- il comprend en outre des moyens à haut-parleur permettant d'informer l'utilisateur sur l'état ventilatoire du patient (i.e. volume insufflé trop important, voies aériennes fermées...) et/ou en cas de déclenchement d'une alarme.
- la carcasse du ventilateur a des dimensions de l'ordre de 30 cm x 30 cm x 15 cm.
- la source de gaz, les moyens de pilotage à microprocesseur, et les moyens d'affichage sont intégrés au ventilateur, c'est-à-dire agencés sur ou dans le ventilateur, en particulier sur ou dans la carcasse du ventilateur.

L'invention va maintenant être mieux comprise grâce à la description détaillée suivante, faite à titre illustratif mais non limitatif, en référence aux figures annexées parmi lesquelles :
Fig. 1 schématise un mode de réalisation d'un ventilateur médical selon l'invention ; et
Fig. 2 schématise l'architecture interne du ventilateur médical de Fig. 1.

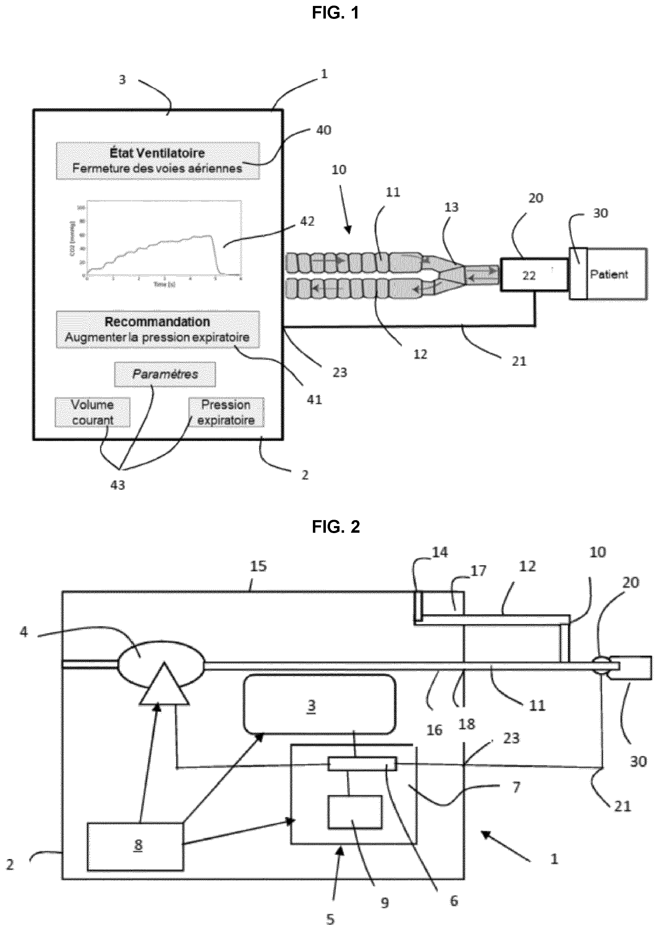

Fig. 1 schématise un mode de réalisation d'un ventilateur médical 1, c'est-à-dire un appareil de ventilation assistée, selon l'invention, conçu pour être utilisé pendant une réanimation cardio-pulmonaire ou RCP, pratiquée par des secouristes sur une personne, appelée patient, en arrêt cardio-pulmonaire.

Ce ventilateur médical 1 permet de délivrer un gaz d'assistance contenant de l'oxygène au patient, tel de l'air ou un mélange air/O₂, pendant que des secouristes ou personnels soignants pratiquent un massage cardiaque sur ledit patient avec alternance de compressions et décompressions thoraciques successives, à une fréquence d'environ 100 à 120 coups/minutes avec une profondeur de compression entre 4 et 6 cm.

Le ventilateur médical 1 comprend une carcasse 2 ou coque externe rigide, par exemple en polymère, contenant, comme schématisé en Fig. 2, différents éléments ou composants assurant le bon fonctionnement du ventilateur 1, en particulier une source de gaz 4, telle une turbine électrique motorisée, aussi appelée micro-soufflante ou compresseur, délivrant le gaz respiratoire à un circuit de ventilation 10, via un circuit de gaz interne 15, tel un conduit ou passage de gaz, et des moyens de pilotage 5 à microprocesseur(s) 6, c'est-à-dire des moyens de traitement de données, telle une carte électronique 7 comprenant un ou plusieurs microprocesseurs 6 mettant en oeuvre un ou des algorithmes ou autres programmes d'ordinateur.

Le ventilateur médical 1 comprend aussi des moyens d'affichage 3 commandés par les moyens de pilotage 5, typiquement un (ou des) écran ou afficheur électronique, par exemple agencé dans l'une des faces de la carcasse 2 ou, selon un autre mode de réalisation (non montré) porté par un bras-support surmontant le ventilateur 1.

La source de gaz 4, qui est une turbine électrique motorisée, alimente en gaz un circuit ventilatoire 10 à double branche 11, 12 permettant d'acheminer les flux gazeux vers le patient ou de récupérer le flux gaz contenant du CO₂ sortant des poumons du patient.

Selon un mode de réalisation non montré et hors invention, le ventilateur 1 ne comporte pas de micro-soufflante interne en tant que source de gaz mais des entrées et conduits d'alimentation en air et oxygène sous pression, typiquement de l'ordre de quelques bar, par exemple entre 2 et 6 bar. Dans ce cas, les prises des capteurs de pression et débit sont par exemple reliées entre la pièce en Y 13 et l'interface patient 30, c'est-à-dire en configuration 'main stream' ou flux principal ; toutefois, on peut aussi les agencer en flux dérivé ou 'side stream', comme précédem ment.

Le circuit ventilatoire 10 comprend une branche inspiratoire 11 et une branche expiratoire 12 agencées en parallèle, telles des tuyaux flexibles en polymère.

Plus précisément, la branche inspiratoire 11 est alimentée par la source de gaz 4 et achemine le gaz respiratoire (i.e. air ou air/O₂) jusqu'au patient, alors que la branche expiratoire 12 permet de convoyer les gaz riches en CO₂ sortant du patient.

La branche expiratoire 12 est reliée à l'atmosphère via un port de sortie 14 afin d'évacuer les gaz riches en CO₂ sortant des poumons du patient, i.e. les gaz dits « expirés ».

Les branches inspiratoire 11 et expiratoire 12 viennent se raccorder l'une et l'autre, mécaniquement et fluidiquement à une pièce de jonction 13 ou pièce en Y, située entre lesdites branches inspiratoire 11 et expiratoire 12 et une interface respiratoire patient 30, tel un masque respiratoire ou une sonde d'intubation trachéale, servant à assurer les échanges gazeux entre le ventilateur 1 et le patient.

La pièce en Y 13 assure donc la continuité fluidique entre, d'une part, les branches inspiratoire 11 et expiratoire 12 et, d'autre part, l'interface respiratoire 30 afin que le gaz respiratoire puisse circuler de la branche inspiratoire 11 vers l'interface respiratoire 30 lors des phases de fourniture de gaz par la turbine 4 et, à l'inverse, de l'interface respiratoire 30 vers la branche expiratoire 12 lorsque le gaz ressort des poumons du patient (sous l'effet de la phase expiratoire de la ventilation et de la phase de compression du massage cardiaque).

Afin de permettre d'opérer des mesures de la teneur ou quantité de CO₂ présent dans le flux gazeux provenant des poumons du patient et expulsé pendant la RCP, c'est-à-dire circulant essentiellement de l'interface respiratoire 30 vers la branche expiratoire 12, il est prévu des moyens de mesure de CO₂ 20, 21 agencés à proximité immédiate de l'interface respiratoire 30, à savoir ici entre l'interface respiratoire 30 et la pièce en Y 13.

Les moyens de mesure de CO₂ 20, 21 comprennent un capteur de CO₂ 20 par exemple intégré à une pièce tubulaire 22, et relié aux moyens de pilotage 5 via une liaison filaire 21, tel un câble électrique, venant se raccorder mécaniquement et électriquement à un connecteur 23 porté par le ventilateur 1, ledit connecteur 23 étant lui-même relié électriquement aux moyens de pilotage du ventilateur 1.

Préférentiellement, le capteur de CO₂ 20 est un dispositif de spectrométrie infra-rouge ou analogue.

Les moyens de mesure de CO₂ 20, 21 sont configurés pour opérer des mesures successives de teneur en CO₂ reflétant la quantité de CO₂ présent dans le flux gazeux, et ensuite fournir ces mesures de teneur en CO₂ (sous forme de signaux ou analogues) aux moyens de pilotage 5, qui les traitent informatiquement au sein du ou des microprocesseurs 6 via un ou des algorithmes adaptés.

Par ailleurs, des moyens de fourniture de courant électrique 8, par exemple une batterie rechargeable ou un cordon de raccordement au secteur (110/220V), alimente les moyens de pilotage 5, la turbine 4 motorisée et les moyens d'affichage 3, ainsi que tous les autres composants nécessitant de l'énergie électrique pour fonctionner.

Préférentiellement, les moyens de pilotage 5, en particulier une carte électronique 7 portant le ou les microprocesseurs 6, peuvent aussi comprendre des moyens de stockage de données 9, telle une mémoire de stockage informatique, par exemple de type EEPROM ou autre.

Les moyens de stockage de données 9 enregistrent notamment les données mesurées pendant la RCP, ainsi que des informations ou paramètres s'affichant sur les moyens d'affichage 3 permettant une analyse à posteriori de ces données ou autres. Ils servent aussi préférentiellement à stocker les différents types de diagnostics d'état ventilatoire et d'état circulatoire, et les différentes recommandations d'action à mener, comme expliqué ci-après.

Par ailleurs, les moyens d'affichage 3 comprennent un afficheur numérique, tel qu'un écran en couleurs, par exemple un écran tactile, permettent d'afficher 42 une (ou des) courbe de suivi, telle une courbe de CO₂, et d'autres informations 40, 41, 43, tels des valeurs, des messages, des icônes d'alarme, les états circulatoire ou ventilatoire du patient..., comme expliqué ci-après.

Le ventilateur médical 1 de l'invention permet de monitorer différents paramètres ou signaux respiratoires, pendant une RCP pratiquée sur le patient P en arrêt cardio-respiratoire, en particulier la teneur en CO₂ du gaz mais aussi préférentiellement le débit gazeux et/ou la pression des voies aériennes, pour en déduire par exemple un volume gazeux, de manière à permettre non seulement aux secouristes de suivre l'évolution de la quantité de CO₂ dans les gaz provenant des poumons du patient P, donc de détecter facilement et rapidement un RACS par exemple, mais aussi et surtout de déterminer et fournir un diagnostic de l'état ventilatoire du patient et éventuellement de son état circulatoire, comme détaillé ci-après.

Pour ce faire, les moyens de mesure de CO₂ 20, 21 sont configurés pour fournir aux moyens de pilotage 5, les (signaux de) mesures successives de teneurs en CO₂, et ceux-ci, en particulier leur (leurs) microprocesseur(s) 6, sont configurés pour traiter ces mesures de teneur en CO₂ et commander ensuite un affichage 40-43 sur les moyens d'affichage 3, tel un écran à affichage numérique, d'informations utiles au personnel soignant, telle une courbe en temps réel de CO₂ obtenue à partir des mesures successives de teneurs en CO₂ successives ayant été traitées mais avant tout d'établir et afficher 40, 41 d'un diagnostic de l'état ventilatoire du patient et éventuellement aussi de son état circulatoire.

Plus précisément, les moyens de pilotage 5 sont configurés pour traiter les valeurs de teneurs en CO₂ mesurée, c'est-à-dire les mesures de teneur en CO₂, pour établir au moins un diagnostic de l'état ventilatoire du patient, à savoir une fermeture des voies aériennes, une distension thoracique et une ventilation cible, et commander ensuite l'affichage 40 de ce diagnostic d'état ventilatoire sur les moyens d'affichage 3.

Afin d'établir ce diagnostic d'état ventilatoire, les moyens de pilotage 5 peuvent avoir aussi recours à d'autres paramètres ou mesures, notamment des mesures de pression et/ou de débit gazeux. Dès lors, on prévoit aussi des moyens de mesure de pression, tel un capteur de pression, pour déterminer la pression du gaz et pour fournir au moins une valeur de pression mesurée aux moyens de pilotage et/ou des moyens de détermination de débit, tel un capteur de débit, pour déterminer le débit du gaz dans le circuit ventilatoire 10 et fournir au moins une valeur de débit de gaz aux moyens de pilotage.

Par exemple, Fig 2 montre des sites possibles d'agencement d'un capteur de débit inspiratoire 16 et d'un capteur de débit expiratoire 17 permettant d'opérer des mesures du débit gazeux dans les branches inspiratoire 11 et expiratoire 12, et par ailleurs d'un capteur de pression 18 permettant d'opérer des mesures de pression dans la branche inspiratoire 11. Ces mesures sont ensuite transmises aux moyens de pilotage 5, par exemple par des liaisons filaires ou autres.

Les moyens de pilotage 5 sont quant à eux configurés pour traiter ces mesures de débit et/ou pression, en plus des valeurs de teneurs en CO₂. Ils peuvent aussi commander un affichage 43 de ces valeurs de débit et/ou de pression, voire un volume de gaz (obtenu par intégration du débit sur une durée donnée), à l'attention des secouristes.

D'une façon générale, le suivi de ces paramètres, en plus de la teneur en CO₂, permet d'établir avant tout un diagnostic de l'état ventilatoire du patient (P), pendant la RCP, à partir de tout ou partie des mesures opérées (i.e. débit, pression, teneur en CO₂....) et de l'afficher 40, et ensuite de déterminer et proposer, i.e. d'afficher 41, une recommandation d'action à mener par le personnel soignant, telle ou les secouristes pratiquant la RCP sur la patient, en fonction de ce diagnostic d'état ventilatoire, et ce, afin de permettre une adaptation en temps réel de la ventilation, voire même du massage cardiaque.

Préférentiellement, le suivi et le traitement de ces paramètres permettent également un diagnostic de l'état circulatoire du patient, c'est-à-dire du massage cardiaque en cours, pendant la RCP, à partir de tout ou partie des mesures opérées (débit, pression, teneur en CO₂...) et de proposer, comme précédemment, une recommandation d'action à mener par le personnel soignant, typiquement le ou les secouristes pratiquant la RCP sur le patient, en fonction de ce diagnostic d'état circulatoire et ce, afin de permettre une adaptation en temps réel de la circulation, c'est-à-dire du massage cardiaque qui est pratiqué sur le patient.

Dans ce but, afin de déterminer la pression du gaz dans le circuit de gaz 10 du ventilateur 1, on prévoit des moyens de mesure de pression 18, tel un capteur de pression, pour mesurer la pression du gaz, en particulier dans la branche inspiratoire 11, et fournir les valeurs de pression mesurées aux moyens de pilotage où ces valeurs de pression sont traitées. Il est entendu que les mesures de pression peuvent être soit des valeurs, soit des signaux, par exemple des tensions, représentatifs ou correspondant à des valeurs. Les moyens de pilotage 5, qui reçoivent la ou les mesures de pression, sont configurés pour la/les traiter et ensuite commander éventuellement un affichage, sur les moyens d'affichage 3, d'une valeur de pression ou d'une représentation graphique d'une ou plusieurs valeurs de pression traitées, par exemple sous forme d'un barre-graphe ou d'une courbe de pression au fil du temps.

De même, afin de déterminer le débit gazeux dans le circuit de gaz 10 du ventilateur 1, on prévoit des moyens de détermination de débit 16, 17 permettant de mesurer et fournir ensuite aux moyens de pilotage, une ou des mesures ou valeurs de débit de gaz correspondant au débit du gaz dans le circuit de gaz 10, en particulier dans la branche inspiratoire 11 et expiratoire 12, par exemple un ou des capteurs de débit massique. Les mesures de débit opérées sont reçues et traitées par les moyens de pilotage 5, en particulier pour calculer un (des) volumes de gaz par intégration des valeurs de débits sur un intervalle de temps donné, par exemple sur 0,5 à 10 secondes.

Les moyens de détermination de débit sont ou comprennent par exemple un capteur de débit massique ou un capteur de pression différentielle ou à perte de charge. Dans ce dernier cas, on prévoit un premier et un second piquage de pression, respectivement, séparés l'un de l'autre par un élément à perte de charge, telle une restriction de passage, agencé dans le circuit ventilatoire 10 et reliés fluidiquement au capteur de pression différentiel via des lignes de mesure de pression.

Ainsi, on peut déterminer un débit de gaz à partir des mesures de pression opérées par le capteur de pression différentiel à partir des pressions provenant des premier et second piquages de pression et acheminées par les lignes de mesure de pression jusqu'aux moyens de pilotage, qui les reçoivent, puis les traitent, en particulier pour calculer un (des) volume de gaz par intégration du débit, comme expliqué ci-avant. Les moyens de pilotage 5, qui reçoivent la ou les mesures de pression et calculent un (des) débit et éventuellement un (des) volume de gaz, sont en outre configurés pour aussi commander un affichage, sur les moyens d'affichage 3, des valeurs de débit ou volume ainsi calculées.

Là encore, les mesures de débit ou de pression de gaz sont soit des valeurs numériques, soit des signaux représentatifs ou correspondant à de telles valeurs, par exemple une courbe de débit (Q).

Plus généralement, selon l'invention, afin de permettre une adaptation en temps réel de la ventilation, voire même du massage cardiaque, donc d'aider les personnels soignants pendant la RCP, les moyens de pilotage 5, telle une carte électronique 7 comprenant un (ou des) microprocesseur(s) 6 mettant en oeuvre un ou des algorithmes, sont configurés pour :
- traiter tout ou partie des valeurs (i.e. mesures) de pression de gaz, de débit de gaz et de teneurs en CO₂,
- établir un diagnostic de l'état ventilatoire et éventuellement de l'état circulatoire du patient P à partir des valeurs (i.e. mesures) traitées, et
- ensuite commander un affichage 40 du diagnostic d'état ventilatoire et éventuellement du diagnostic d'état circulatoire sur les moyens d'affichage 3, tel un écran numérique, de préférence à affichage en couleurs, par exemple un écran tactile.

Plus précisément, le diagnostic d'état ventilatoire est typiquement une fermeture des voies aériennes, une distension thoracique (donc un volume ventilatoire trop élevé) ou une ventilation cible (i.e. adaptée) du patient qui est suffisante pour empêcher toute fermeture de ses voies aériennes mais insuffisante pour conduire à toute distension thoracique), ou autre.

Optionnellement, le diagnostic d'état circulatoire peut comprendre aussi une indication de la manière dont s'effectue le massage cardiaque, par exemple massage trop fort ou trop faible (i.e. insuffisant), ou massage trop rapide ou trop lent, ou autre.

Les moyens de pilotage 5 sont en outre configurés pour déterminer une recommandation d'action à mener par le personnel soignant en fonction du diagnostic d'état ventilatoire et/ou circulatoire qui a été établi, et commander aussi un affichage 41 de la recommandation d'action à mener sur les moyens d'affichage 3.

Par exemple, la recommandation d'action à mener et qui s'affiche 41, laquelle est déterminée à partir :
- du diagnostic d'état ventilatoire peut être choisie parmi une augmentation de la pression positive (aussi appelée pression expiratoire positive), une baisse du volume courant ou une continuité (i.e. maintien sans changement) de la ventilation, ou autre, et/ou
- du diagnostic d'état circulatoire peut être choisie parmi une augmentation de la force de compression du massage cardiaque, une diminution de la force de compression du massage cardiaque, une augmentation de la fréquence du massage cardiaque et une diminution de la fréquence du massage cardiaque, ou autre.

Les différents types de diagnostics d'état ventilatoire et d'état circulatoire, et les différentes recommandations d'action à mener sont préenregistrés, c'est-à-dire mémorisés, au sein de moyens de mémorisation 9, par exemple agencés au sein des moyens de pilotage 5, typiquement un carte mémoire ou analogue agencée sur une ou la carte électronique 7.

Les moyens de pilotage 5 sont donc configurés pour aller retrouver au sein des moyens de mémorisation 9, le type de diagnostics d'état ventilatoire et éventuellement d'état circulatoire, et la recommandation d'action à mener appropriés, c'est-à-dire correspondant aux paramètres ayant été mesurés, typiquement les valeurs de teneur en CO₂ et préférentiellement de pression de gaz et de débit de gaz, voire d'autres paramètres si nécessaire, tel que le volume gazeux. Une fois retrouvés dans les moyens de mémorisation 9, le diagnostic d'état ventilatoire et/ou circulatoire, et la recommandation d'action à mener peuvent être affichés 40, 41 sur les moyens d'affichage 3, comme illustré en Fig. 1.

De plus, dans le cadre de l'invention, afin d'améliorer ou compléter le suivi de la teneur en CO₂ du gaz, le ventilateur médical 1 selon l'invention peut être configuré pour suivre et afficher aussi sur les moyens d'affichage 3, un et préférentiellement plusieurs autres paramètres ventilatoires 43, tels que le débit du gaz, notamment le débit expiratoire du patient, et la pression gazeuse reflétant la pression dans les voies aériennes du patient, voire le volume de gaz qui est l'intégrale du débit sur une durée donnée.

Plus généralement, selon l'invention, le monitorage des différents paramètres ventilatoires susmentionnés permet aux moyens de pilotage d'établir un diagnostic d'état ventilatoire et préférentiellement circulatoire du patient, de les afficher 40 et ensuite de déterminer et afficher 41 une recommandation d'action à mener, ce qui constitue une aide très importante pour les personnels soignants lors des RCP, leur permettant de savoir si la RCP si déroule efficacement ou si des adaptations doivent être opérées.

Par ailleurs, le ventilateur médical 1 de l'invention permet aussi d'opérer un suivi et un affichage 42 sous forme d'une courbe en temps réel (ou d'une autre représentation adaptée) de la teneur en CO₂ des gaz sortant des poumons du patient (i.e. capnométrie) pendant la RCP, de manière à encore améliorer le suivi du déroulé de la RCP étant donné qu'une telle courbe de CO₂ donne aux secouristes une indication de bonne ou mauvaise intubation et permet en outre de mesurer la fréquence ventilatoire. Elle inclut aussi une valeur pronostic du patient (i.e. survie ou décès) et facilite la détection du retour à une activité circulatoire spontanée ou RACS et ce, malgré la présence d'oscillations de teneur en CO₂ dans le flux gazeux, provoquées par le massage cardiaque.

Bien entendu, le ventilateur médical 1 de l'invention peut aussi comprendre un ou plusieurs boutons ou touches de sélection (non montrés) actionnables par l'utilisateur, par exemple pour allumer ou pour éteindre le ventilateur médical 1 (on/off), pour opérer des sélections ou des choix, pour acquitter des alarmes, pour permettre à l'utilisateur de configurer le ventilateur 1....

De même, on peut aussi prévoir sur le ventilateur 1, des moyens de connectivité (non montrés) permettant d'échanger ou de télécharger des données, par exemple un port USB ou autre, ou encore des composants habituels permettant le bon fonctionnement du ventilateur 1, comme une ou des valves de contrôle de la circulation des flux gazeux, un ou des clapets anti-retour....

Grâce aux moyens de mémorisation permettant de mémoriser les diagnostics d'état ventilatoire, circulatoire et des recommandations d'actions à mener, le ventilateur médical 1 de l'invention peut aussi générer un rapport de l'intervention.

De plus, le ventilateur médical 1 de l'invention peut aussi comprendre des moyens d'alarme sonore et/ou visuelle configurés pour déclencher une alarme, notamment lorsque l'on détecte une pression des voies aériennes trop importante, un débranchement patient, un volume insufflé trop important, des voies aériennes fermées ou autres. Les moyens d'alarme sonore et/ou visuelle peuvent coopérer ou être inclus dans les moyens de pilotage 5.

Les moyens d'alarme sonore et/ou visuelle comprennent par exemple des moyens à haut-parleur permettant d'informer de manière sonore, i.e. déclenchement d'un signal audible, l'utilisateur sur l'état ventilatoire du patient (i.e. volume insufflé trop important, voies aériennes fermées...) et/ou en cas de déclenchement d'une alarme.

D'une façon générale, l'invention permet d'opérer un monitorage efficace d'une ventilation assistée délivrée par l'appareil d'assistance ventilatoire ou de ventilation assistée ou ventilateur médical 1 de l'invention, pendant une réanimation cardio-pulmonaire (RCP) pratiquée sur une personne arrêt cardio-pulmonaire.

## Revendications

1. Ventilateur médical (1) adapté à la mise en oeuvre d'une ventilation assistée pendant une réanimation cardio-pulmonaire (RCP) d'un patient, comprenant :
- une source de gaz (4),
- un circuit ventilatoire (10) comprenant une branche inspiratoire (11) alimentée en gaz par la source de gaz (4), et une branche expiratoire (12) venant se raccorder l'une et l'autre à une pièce de jonction (13),
- une interface respiratoire (30) raccordée à la pièce de jonction (13) et en communication fluidique avec le circuit ventilatoire (10),
- des moyens de pilotage (5) à microprocesseur (6),
- des moyens de mesure de CO₂ (20, 21) configurés pour opérer des mesures successives de teneur en CO₂ dans le flux gazeux et fournir lesdites mesures de teneur en CO₂ aux moyens de pilotage (5), lesdits moyens de pilotage (5) traitant lesdites mesures de teneur en CO₂, et
- des moyens d'affichage (3) commandés par lesdits moyens de pilotage (5),
et dans lequel :
- les moyens de pilotage (5) sont en outre configurés pour :
i) traiter au moins une partie des valeurs de teneurs en CO₂ pour établir au moins un diagnostic de l'état ventilatoire du patient choisi parmi une fermeture des voies aériennes, une distension thoracique et une ventilation cible, et
ii) commander un affichage (40) dudit au moins un diagnostic d'état ventilatoire sur les moyens d'affichage (9),
- et les moyens d'affichage (3) sont configurés pour afficher (40) ledit au moins un diagnostic d'état ventilatoire, et dans lequel :
- la source de gaz (4) comprend une turbine motorisée commandée par les moyens de pilotage (5), et
**caractérisé en ce que**:
- les moyens de pilotage (5) sont en outre configurés pour :
i) déterminer au moins une recommandation d'action à mener par le personnel soignant en fonction dudit au moins un diagnostic d'état ventilatoire, et
ii) commander un affichage (41) de ladite au moins une recommandation d'action à mener sur les moyens d'affichage (3), et
- les moyens d'affichage (3) sont configurés pour afficher (41) ladite au moins une recommandation d'action à mener.

2. Ventilateur selon la revendication 1, **caractérisé en ce qu'**il comprend en outre :
- des moyens de mesure de pression pour déterminer au moins une pression du gaz et pour fournir ladite au moins une valeur de pression mesurée aux moyens de pilotage et
- des moyens de détermination de débit pour déterminer au moins une valeur de débit de gaz correspondant au débit du gaz dans le circuit ventilatoire (10) et fournir ladite au moins une valeur de débit de gaz auxdits moyens de pilotage.

3. Ventilateur selon la revendication 2, **caractérisé en ce que** les moyens de pilotage (5) sont configurés pour traiter lesdites au moins une valeur de pression et de débit mesurées pour établir ledit au moins un diagnostic de l'état ventilatoire du patient ou pour confirmer ledit au moins un diagnostic établi par les moyens de pilotage (5).

4. Ventilateur selon la revendication 1, **caractérisé en ce que** les moyens de mesure de CO₂ (20, 21) comprennent un capteur de CO₂ (20) agencé entre l'interface respiratoire (30) et la pièce de jonction (13), et relié aux moyens de pilotage via une liaison filaire (21).

5. Ventilateur selon la revendication 1, **caractérisé en ce que** les moyens d'affichage sont en outre configurés pour afficher (42) une courbe de CO₂, de préférence en temps réel, à partir des mesures de teneur en CO₂ traitées par les moyens de pilotage.

6. Ventilateur selon la revendication 1, **caractérisé en ce que** :
- les moyens de pilotage (5) sont en outre configurés pour établir un diagnostic d'état circulatoire du patient à partir d'au moins une partie des mesures traitées et pour commander un affichage dudit diagnostic d'état circulatoire sur les moyens d'affichage (3), et
- les moyens d'affichage (3) étant configurés pour afficher ledit diagnostic d'état circulatoire.

7. Ventilateur selon les revendications 2 et 6, **caractérisé en ce que** les moyens de pilotage (5) sont configurés pour traiter au moins une partie des mesures de teneurs en CO₂ et de pression et/ou de débit de gaz pour établir ledit au moins un diagnostic d'état ventilatoire et/ou d'état circulatoire.

8. Ventilateur selon les revendications 1 et 6, caractérisé ce que les moyens de pilotage (5) sont en outre configurés pour déterminer ladite recommandation d'action à mener en fonction desdits diagnostics d'état ventilatoire et d'état circulatoire.

9. Ventilateur selon la revendication 1, **caractérisé en ce que** ladite au moins une recommandation d'action à mener est choisie parmi :
- une augmentation de la pression positive, une baisse du volume courant ou une continuité de la ventilation, et/ou
- une augmentation ou une diminution de la force de compression du massage cardiaque, et
- une augmentation ou une diminution de la fréquence du massage cardiaque.

10. Ventilateur selon l'une des revendications 1 et 6, **caractérisé en ce qu'**il comprend en outre des moyens de mémorisation (9) pour mémoriser les diagnostics d'état ventilatoire, les diagnostics d'état circulatoire et les recommandations d'action à mener.

11. Ventilateur selon la revendication 10, **caractérisé en ce que** les moyens de pilotage (5) sont en outre configurés pour aller retrouver au sein des moyens de mémorisation (9), le diagnostic d'état ventilatoire, le diagnostic d'état circulatoire et/ou la recommandation d'action à mener à afficher sur les moyens d'affichage (3).

12. Ventilateur selon les revendications 1, 2, 6 et 10, **caractérisé en ce que** les moyens de pilotage (5) sont configurés pour établir au moins un diagnostic de l'état ventilatoire et/ou circulatoire du patient et pour proposer une recommandation d'action à mener en allant retrouver au sein des moyens de mémorisation (9), le diagnostic d'état ventilatoire, le diagnostic d'état circulatoire et/ou la recommandation d'action à mener à afficher sur les moyens d'affichage (3) correspondant aux valeurs de teneurs en CO₂, de débit et/ou de pression ayant été traitées par lesdits moyens de pilotage (5).

13. Ventilateur selon l'une des revendications précédentes, **caractérisé en ce que** la source de gaz (4), les moyens de pilotage (5) et les moyens d'affichage (3) sont intégrés au ventilateur (1), en étant agencés sur ou dans la carcasse (2) du ventilateur (1).

14. Ventilateur selon l'une des revendications précédentes, **caractérisé en ce que** les moyens de pilotage (5) comprennent au moins une carte électronique comprenant au moins un microprocesseur mettant en oeuvre un ou plusieurs algorithmes, de préférence la carte électronique comprend les moyens de stockage de données (9).

15. Ventilateur selon l'une des revendications précédentes, **caractérisé en ce que** les moyens d'affichage (3) comprennent un écran d'affichage.

## Patentansprüche

1. Medizinisches Beatmungsgerät (1), welches für die Durchführung einer assistierten Beatmung während einer kardiopulmonalen Wiederbelebung (CPR) eines Patienten eingerichtet ist, umfassend:
- eine Gasquelle (4),
- einen Beatmungskreislauf (10), der einen von der Gasquelle (4) mit Gas versorgten Einatmungszweig (11) und einen Ausatmungszweig (12) umfasst, die beide an ein Verbindungsstück (13) angeschlossen sind,
- eine respiratorische Schnittstelle (30), die an das Verbindungsstück (13) angeschlossen ist und mit dem Beatmungskreislauf (10) in Fluidverbindung steht,
- Steuerungsmittel (5) mit einem Mikroprozessor (6),
- CO₂-Messmittel (20, 21) die dafür ausgelegt sind, aufeinander folgende Messungen des CO₂-Gehalts im Gasstrom durchzuführen und die Messwerte des CO₂-Gehalts an die Steuerungsmittel (5) zu übermitteln, wobei die Steuerungsmittel (5) die Messwerte des CO₂-Gehalts verarbeiten, und
- Anzeigemittel (3), die von den Steuerungsmitteln (5) gesteuert werden,
und wobei:
- die Steuerungsmittel (5) außerdem dafür ausgelegt sind:
i) wenigstens einen Teil der Werte des CO₂-Gehalts zu verarbeiten, um mindestens eine Diagnose des Beatmungszustands des Patienten zu erstellen, die aus einem Verschluss der Atemwege, einer Thoraxdistension und einer Zielbeatmung ausgewählt ist, und
ii) eine Anzeige (40) der mindestens einen Diagnose des Beatmungszustands auf den Anzeigemitteln (9) zu steuern,
- und die Anzeigemittel (3) dafür ausgelegt sind, die mindestens eine Diagnose des Beatmungszustands anzuzeigen (40),
und wobei:
- die Gasquelle (4) eine motorisierte Turbine umfasst, die von den Steuerungsmitteln (5) gesteuert wird, und **dadurch gekennzeichnet, dass**:
- die Steuerungsmittel (5) außerdem dafür ausgelegt sind:
i) mindestens eine Empfehlung einer vom Pflegepersonal auszuführenden Handlung in Abhängigkeit von der mindestens einen Diagnose des Beatmungszustands zu bestimmen, und
ii) eine Anzeige (41) der mindestens einen Empfehlung einer auszuführenden Handlung auf den Anzeigemitteln (3) zu steuern, und
- die Anzeigemittel (3) dafür ausgelegt sind, die mindestens einen Empfehlung einer auszuführenden Handlung anzuzeigen (41).

2. Beatmungsgerät nach Anspruch 1, **dadurch gekennzeichnet, dass** es außerdem umfasst:
- Druckmessmittel zum Bestimmen mindestens eines Drucks des Gases und zum Übermitteln des mindestens einen gemessenen Druckwertes an die Steuerungsmittel und
- Mittel zur Durchflussbestimmung zum Bestimmen mindestens eines dem Durchfluss des Gases im Beatmungskreislauf (10) entsprechenden Gasdurchflusswertes und Übermitteln des mindestens einen Gasdurchflusswertes an die Steuerungsmittel.

3. Beatmungsgerät nach Anspruch 2, **dadurch gekennzeichnet, dass** die Steuerungsmittel (5) dafür ausgelegt sind, den mindestens einen Druckwert und den mindestens einen Durchflusswert, die gemessen wurden, zu verarbeiten, um die mindestens eine Diagnose des Beatmungszustands des Patienten zu erstellen oder um die von den Steuerungsmitteln (5) erstellte mindestens eine Diagnose zu bestätigen.

4. Beatmungsgerät nach Anspruch 1, **dadurch gekennzeichnet, dass** die CO₂-Messmittel (20, 21) einen CO₂-Sensor (20) umfassen, der zwischen der respiratorischen Schnittstelle (30) und dem Verbindungsstück (13) angeordnet ist und über eine Drahtverbindung (21) mit den Steuerungsmitteln verbunden ist.

5. Beatmungsgerät nach Anspruch 1, **dadurch gekennzeichnet, dass** die Anzeigemittel außerdem dafür ausgelegt sind, ausgehend von den von den Steuerungsmitteln verarbeiteten Messwerten des CO₂-Gehalts eine CO₂-Kurve anzuzeigen (42), vorzugsweise in Echtzeit.

6. Beatmungsgerät nach Anspruch 1, **dadurch gekennzeichnet, dass**:
- die Steuerungsmittel (5) außerdem dafür ausgelegt sind, aus wenigstens einem Teil der verarbeiteten Messwerte eine Diagnose des Kreislaufzustands des Patienten zu erstellen und eine Anzeige der Diagnose des Kreislaufzustands auf den Anzeigemitteln (3) zu steuern, und
- die Anzeigemittel (3) dafür ausgelegt sind, die Diagnose des Kreislaufzustands anzuzeigen.

7. Beatmungsgerät nach den Ansprüchen 2 und 6, **dadurch gekennzeichnet, dass** die Steuerungsmittel (5) dafür ausgelegt sind, wenigstens einen Teil der Messwerte des CO₂-Gehalts und des Drucks und/oder des Gasdurchflusses zu verarbeiten, um die mindestens eine Diagnose des Beatmungszustands und/oder des Kreislaufzustands zu erstellen.

8. Beatmungsgerät nach den Ansprüchen 1 und 6, **dadurch gekennzeichnet, dass** die Steuerungsmittel (5) außerdem dafür ausgelegt sind, die Empfehlung einer auszuführenden Handlung in Abhängigkeit von den Diagnosen des Beatmungszustands und des Kreislaufzustands zu bestimmen.

9. Beatmungsgerät nach Anspruch 1, **dadurch gekennzeichnet, dass** die mindestens eine Empfehlung einer auszuführenden Handlung ausgewählt ist aus:
- einer Erhöhung des positiven Drucks, einer Verringerung des Tidalvolumens oder einer unveränderten Fortsetzung der Beatmung, und/oder
- einer Erhöhung oder einer Verringerung der Druckkraft der Herzmassage und
- einer Erhöhung oder einer Verringerung der Frequenz der Herzmassage.

10. Beatmungsgerät nach einem der Ansprüche 1 und 6, **dadurch gekennzeichnet, dass** es außerdem Speichermittel (9) zum Speichern der Diagnosen des Beatmungszustands, der Diagnosen des Kreislaufzustands und der Empfehlungen einer auszuführenden Handlung umfasst.

11. Beatmungsgerät nach Anspruch 10, **dadurch gekennzeichnet, dass** die Steuerungsmittel (5) außerdem dafür ausgelegt sind, aus den Speichermitteln (9) die Diagnose des Beatmungszustands, die Diagnose des Kreislaufzustands und/oder die Empfehlung einer auszuführenden Handlung abzurufen, die auf den Anzeigemitteln (3) anzuzeigen ist bzw. sind.

12. Beatmungsgerät nach den Ansprüchen 1, 2, 6 und 10, **dadurch gekennzeichnet, dass** die Steuerungsmittel (5) dafür ausgelegt sind, mindestens eine Diagnose des Beatmungszustands und/oder Kreislaufzustands des Patienten zu erstellen und eine Empfehlung einer auszuführenden Handlung vorzuschlagen, indem sie aus den Speichermitteln (9) die auf den Anzeigemitteln (3) anzuzeigende Diagnose des Beatmungszustands, Diagnose des Kreislaufzustands und/oder Empfehlung einer auszuführenden Handlung abrufen, die den Werten des CO₂-Gehalts, des Durchflusses und/oder des Drucks entspricht bzw. entsprechen, die von den Steuerungsmitteln (5) verarbeitet worden sind.

13. Beatmungsgerät nach einem der vorhergehenden Ansprüche, **dadurch gekennzeichnet, dass** die Gasquelle (4), die Steuerungsmittel (5) und die Anzeigemittel (3) in das Beatmungsgerät (1) integriert sind, indem sie auf oder in dem Gehäuse (2) des Beatmungsgeräts (1) angeordnet sind.

14. Beatmungsgerät nach einem der vorhergehenden Ansprüche, **dadurch gekennzeichnet, dass** die Steuerungsmittel (5) mindestens eine Leiterplatte umfassen, die mindestens einen Mikroprozessor umfasst, der einen oder mehrere Algorithmen ausführt, wobei die Leiterplatte vorzugsweise die Datenspeichermittel (9) umfasst.

15. Beatmungsgerät nach einem der vorhergehenden Ansprüche, **dadurch gekennzeichnet, dass** die Anzeigemittel (3) einen Anzeigebildschirm umfassen.

## Claims

1. Medical ventilator (1) designed for the implementation of assisted ventilation during cardiopulmonary resuscitation (CPR) of a patient, comprising:
- a gas source (4),
- a ventilatory circuit (10) comprising an inspiratory branch (11) supplied with gas via the gas source (4), and an expiratory branch (12), which are both connected to a junction piece (13),
- a respiratory interface (30) connected to the junction piece (13) and in fluidic communication with the ventilatory circuit (10),
- control means (5) with microprocessor (6),
- CO₂ measurement means (20, 21) configured to carry out successive measurements of CO₂ content in the gas flow and provide said CO₂ content measurements to the control means (5), said control means (5) processing said CO₂ content measurements, and
- display means (3) controlled by said control means (5),
and wherein:
- the control means (5) are also configured to:
i) process at least some of the CO₂ content values so as to establish at least one diagnosis of the ventilatory status of the patient chosen from closure of the airways, thoracic distension and target ventilation, and
ii) order display (40) of said at least one ventilatory status diagnosis on the display means (9),
- and the display means (3) are configured to display (40) said at least one ventilatory status diagnosis, and wherein:
- the gas source (4) comprises a motorized turbine controlled by the control means (5), and **characterized in that**:
- the control means (5) are also configured to:
i) determine at least one recommendation of action to be taken by the medical personnel as a function of said at least one ventilatory status diagnosis, and
ii) order display (41) of said at least one recommendation of action to be taken on the display means (3), and
- the display means (3) are configured to display (41) said at least one recommendation of action to be taken.

2. Ventilator according to Claim 1, **characterized in that** it also comprises:
- pressure measurement means for determining at least one pressure of the gas and for providing said at least one measured pressure value to the control means and
- flow rate determination means for determining at least one gas flow rate value corresponding to the flow rate of the gas in the ventilatory circuit (10) and providing said at least one gas flow rate value to said control means.

3. Ventilator according to Claim 2, **characterized in that** the control means (5) are configured to process said at least one measured pressure value and measured flow rate value so as to establish said at least one diagnosis of the ventilatory status of the patient or so as to confirm said at least one diagnosis established by the control means (5).

4. Ventilator according to Claim 1, **characterized in that** the CO₂ measurement means (20, 21) comprise a CO₂ sensor (20) arranged between the respiratory interface (30) and the junction piece (13), and connected to the control means via a wired connection (21).

5. Ventilator according to Claim 1, **characterized in that** the display means are also configured to display (42) a CO₂ curve, preferably in real time, on the basis of the CO₂ content measurements processed by the control means.

6. Ventilator according to Claim 1, **characterized in that**:
- the control means (5) are also configured to establish a circulatory status diagnosis of the patient on the basis of at least some of the processed measurements and to order display of said circulatory status diagnosis on the display means (3), and
- the display means (3) are configured to display said circulatory status diagnosis.

7. Ventilator according to Claims 2 and 6, **characterized in that** the control means (5) are configured to process at least some of the CO₂ content and pressure and/or gas flow rate measurements so as to establish said at least one ventilatory status and/or circulatory status diagnosis.

8. Ventilator according to Claims 1 and 6, **characterized in that** the control means (5) are also configured to determine said recommendation of action to be taken as a function of said ventilatory status and circulatory status diagnoses.

9. Ventilator according to Claim 1, **characterized in that** said at least one recommendation of action to be taken is chosen from:
- an increase in the positive pressure, a drop in the tidal volume or a continuity of the ventilation, and/or
- an increase or a decrease in the compression force of the cardiac massage, and
- an increase or a decrease in the frequency of the cardiac massage.

10. Ventilator according to either of Claims 1 and 6, **characterized in that** it also comprises memorization means (9) for memorizing the ventilatory status diagnoses, the circulatory status diagnoses and the recommendations of action to be taken.

11. Ventilator according to Claim 10, **characterized in that** the control means (5) are also configured to retrieve, within the memorization means (9), the ventilatory status diagnosis, the circulatory status diagnosis and/or the recommendation of action to be taken to be displayed on the display means (3).

12. Ventilator according to Claims 1, 2, 6 and 10, **characterized in that** the control means (5) are configured to establish at least one diagnosis of the ventilatory and/or circulatory status of the patient and to propose a recommendation of action to be taken by retrieving, within the memorization means (9), the ventilatory status diagnosis, the circulatory status diagnosis and/or the recommendation of action to be taken to be displayed on the display means (3) corresponding to the CO₂ content, flow rate and/or pressure values that have been processed by said control means (5).

13. Ventilator according to one of the preceding claims, **characterized in that** the gas source (4), the control means (5) and the display means (3) are integrated in the ventilator (1), being arranged on or in the shell (2) of the ventilator (1).

14. Ventilator according to one of the preceding claims, **characterized in that** the control means (5) comprise at least one circuit board comprising at least one microprocessor implementing one or more algorithms, preferably the circuit board comprises the data storage means (9).

15. Ventilator according to one of the preceding claims, **characterized in that** the display means (3) comprise a display screen.
